# EUROPEAN PATENT APPLICATION

(11) **EP 1 103 608 A1**
(43) Date of publication of application: **30.05.2001**
(21) Application number: 99402926.2
(22) Date of filing: 25.11.1999
(51) Int. Cl.: C12N 15/12, C07K 14/47, C07K 16/18, A61K 38/17, C12Q 1/68, G01N 33/68

(54) **CIDE-like protein, its isolation and uses**

(71) Applicant: SANOFI-SYNTHELABO, 75013 Paris (FR)
(72) Inventor: Grailhe, Patrick, 92160 Anthony (FR); O'Brien Donogh, 91380 Chilly Mazarin (FR)
(74) Representative: Thouret-Lemaitre, Elisabeth

(57) **Abstract**

HLYAL47 polypeptides and polynucleotides and methods for producing such polypeptides by recombinant techniques are disclosed. Also disclosed are methods for utilizing HLYAL47 polypeptides and polynucleotides in the design of protocols for the treatment of several pathologies, and diagnostic assays for such conditions.

SEQUENCE LISTING

<110> SANOFI-SYNTHELABO

<120> CIDE-like protein, its isolation and uses.

<130> HLYAL47

<140>
<141>

<160> 2

<170> PatentIn Ver. 2.1

<210> 1
<211> 1261
<212> DNA
<213> Homo sapiens

<220>
<221> CDS
<222> (133)..(792)

<220>
<221> 5'UTR
<222> (1)..(132)

<220>
<221> 3'UTR
<222> (793)..(1261)

<400> 1
tcgacccacg cgtccgctcc gaccaaggag gaaggaaaga gcaggtttgt aacagaaaac 60

accactaaag ccccagcaca ggagagaacc acccagccca gaagttccag ggaaggaact 120

ctccggtcca cc atg gag tac ctc tca gct ctg aac ccc agt gac tta ctc 171
              Met Glu Tyr Leu Ser Ala Leu Asn Pro Ser Asp Leu Leu
                1               5                  10

agg tca gta tct aat ata agc tcg gag ttt gga cgg agg gtc tgg acc   219

Arg Ser Val Ser Asn Ile Ser Ser Glu Phe Gly Arg Arg Val Trp Thr
    15                  20                  25

tca gct cca cca ccc cag cga cct ttc cgt gtc tgt gat cac aag cgg 267
Ser Ala Pro Pro Pro Gln Arg Pro Phe Arg Val Cys Asp His Lys Arg
30                  35                  40                  45

acc atc cgg aaa ggc ctg aca gct gcc acc cgc cag gag ctg cta gcc 315
Thr Ile Arg Lys Gly Leu Thr Ala Ala Thr Arg Gln Glu Leu Leu Ala
                50                  55                  60

aaa gca ttg gag acc cta ctg ctg aat gga gtg cta acc ctg gtg cta 363
Lys Ala Leu Glu Thr Leu Leu Leu Asn Gly Val Leu Thr Leu Val Leu
            65                  70                  75

gag gag gat gga act gca gtg gac agt gag gac ttc ttc cag ctg ctg 411
Glu Glu Asp Gly Thr Ala Val Asp Ser Glu Asp Phe Phe Gln Leu Leu
        80                  85                  90

gag gat gac acg tgc ctg atg gtg ttg cag tct ggt cag agc tgg agc 459
Glu Asp Asp Thr Cys Leu Met Val Leu Gln Ser Gly Gln Ser Trp Ser
    95                  100                 105

cct aca agg agt gga gtg ctg tca tat ggc ctg gga cgg gag agg ccc 507
Pro Thr Arg Ser Gly Val Leu Ser Tyr Gly Leu Gly Arg Glu Arg Pro
110                 115                 120                 125

aag cac agc aag gac atc gcc cga ttc acc ttt gac gtg tac aag caa 555
Lys His Ser Lys Asp Ile Ala Arg Phe Thr Phe Asp Val Tyr Lys Gln
                130                 135                 140

aac cct cga gac ctc ttt ggc agc ctg aat gtc aaa gcc aca ttc tac 603
Asn Pro Arg Asp Leu Phe Gly Ser Leu Asn Val Lys Ala Thr Phe Tyr
            145                 150                 155

ggg ctc tac tct atg agt tgt gac ttt caa gga ctt ggc cca aag aaa 651
Gly Leu Tyr Ser Met Ser Cys Asp Phe Gln Gly Leu Gly Pro Lys Lys
        160                 165                 170

gta ctc agg gag ctc ctt cgt tgg acc tcc aca ctg ctg caa ggc ctg 699
Val Leu Arg Glu Leu Leu Arg Trp Thr Ser Thr Leu Leu Gln Gly Leu
    175                 180                 185

ggc cat atg ttg ctg gga att tcc tcc acc ctt cgt cat gca gtg gag    747
Gly His Met Leu Leu Gly Ile Ser Ser Thr Leu Arg His Ala Val Glu
190                 195                 200                 205

ggg gct gag cag tgg cag cag aag ggc cgc ctc cat tcc tac taa         792
Gly Ala Glu Gln Trp Gln Gln Lys Gly Arg Leu His Ser Tyr
            210                 215                 220

ggggctctga gcttctgccc ccagaatcat tccaaccgac ccactgcaaa gactatgaca 852

gcatcaaatt tcaggacctg cagacagtac aggctagata acccacccaa tttccccact 912

gtcctctgat cccctcgtga cagaaccttt cagcataacg cctcacatcc caagtctata 972

cccttacctg aagaatgctg ttctttccta gccacctttc tagcctccca cttgccctga 1032

aaggccaaga tcaagatgtc ccccaggcat cttgatccca gcctgactgc tgctacatct 1092

aatcccctac caatgcctcc tgtccctaaa ctccccagca tactgatgac agccctctct 1152

gactttacct tgagatctgt cttcataccc ttcccctcaa actaacaaaa acatttccaa 1212

taaaaatatc aaatatttac caaaaaaaaa aaaaaaaaaa aaaaagggc             1261

<210> 2
<211> 219
<212> PRT
<213> Homo sapiens

<400> 2
Met Glu Tyr Leu Ser Ala Leu Asn Pro Ser Asp Leu Leu Arg Ser Val
  1               5                  10                  15

Ser Asn Ile Ser Ser Glu Phe Gly Arg Arg Val Trp Thr Ser Ala Pro
             20                  25                  30

Pro Pro Gln Arg Pro Phe Arg Val Cys Asp His Lys Arg Thr Ile Arg
         35                  40                  45

Lys Gly Leu Thr Ala Ala Thr Arg Gln Glu Leu Leu Ala Lys Ala Leu

    50                  55                  60

Glu Thr Leu Leu Leu Asn Gly Val Leu Thr Leu Val Leu Glu Glu Asp
65                  70                  75                  80

Gly Thr Ala Val Asp Ser Glu Asp Phe Phe Gln Leu Leu Glu Asp Asp
                85                  90                  95

Thr Cys Leu Met Val Leu Gln Ser Gly Gln Ser Trp Ser Pro Thr Arg
            100                 105                 110

Ser Gly Val Leu Ser Tyr Gly Leu Gly Arg Glu Arg Pro Lys His Ser
        115                 120                 125

Lys Asp Ile Ala Arg Phe Thr Phe Asp Val Tyr Lys Gln Asn Pro Arg
    130                 135                 140

Asp Leu Phe Gly Ser Leu Asn Val Lys Ala Thr Phe Tyr Gly Leu Tyr
145                 150                 155                 160

Ser Met Ser Cys Asp Phe Gln Gly Leu Gly Pro Lys Lys Val Leu Arg
                165                 170                 175

Glu Leu Leu Arg Trp Thr Ser Thr Leu Leu Gln Gly Leu Gly His Met
            180                 185                 190

Leu Leu Gly Ile Ser Ser Thr Leu Arg His Ala Val Glu Gly Ala Glu
        195                 200                 205

Gln Trp Gln Gln Lys Gly Arg Leu His Ser Tyr
    210                 215

## Description

The invention relates to newly identified polynucleotides, polypeptides encoded by them, to the use of such polynucleotides and polypeptides, and to their production. More particularly, the polynucleotides and corresponding polypeptides of the present invention are cellular proteins, hereinafter referred to as human CIDE B.

Programmed cell death or apoptosis is a physiological process that eliminates unnecessary cells to maintain tissue homeostasis during development. Dysregulation of this highly controlled process is now recognized as being involved in several pathologies like neurodegenerative diseases, cancer, hematological diseases (leukemia, bone marrow failure), cardiovascular diseases (coronary heart disease, ischemia / reperfusion injury) and inflammation-related disorders.

DNA fragmentation is the most recognized hallmark of apoptosis, occurring at the final irreversible step of cell death. Cleavage of chromatin into nucleosomal fragments is processed by endonucleases that are activated upstream of the cascade of caspases. DNA Fragmentation Factor (DFF), the most characterized endonuclease, is a heterodimeric protein composed of DFF40 (40 kDa) and DFF45 (45 kDa) (Lui X. et al. Cell 89: 175-84, 1997). DFF40 is an endonuclease previously identified in mouse as CAD (Caspase-3 Activated DNAse) by Enari *et al.* (Nature 391:43-50, 1998) whereas its human counterpart was also called CPAN (caspase activated nuclease) by Hallenbeck *et al.* (Cur. Biol. 8:537-40, 1998). DFF40 is present in cytosol as a latent complex, with its inhibitor DFF45 that stabilizes DFF40 in an inactive state. The mouse counterpart to DFF45, ICAD, was identified as a long (ICAD_{L}) and a short (ICAD_{S}) isoform (Sakahira *et al.* Nature 391:96-9, 1998). Upon cleavage by caspase 3 (and probably caspase 6) DFF45 releases DFF40 that becomes active, translocates the nucleus, triggers chromatin condensation and internucleosomal DNA fragmentation. DFF represents a direct link between activated caspase and DNA fragmentation. Expression of recombinant DFF40 protein has demonstrated that DFF45 is necessary to properly fold and stabilize DFF40, acting like a "chaperon". DFF40 is more active on chromatin DNA than on naked DNA, suggesting that nuclear proteins are required as cofactors to become fully active. Chromatin-associated proteins like histone H1 and the nuclear factor HMG2 (High Mobility Group protein 2) enhance nuclease activity of DFF40 (Yon Toh S. et al. Biochem. Biophys. Res. Com. 1998, 250, 598-601). Studies in cell free systems have demonstrated that DFF45, when present in cytosol from healthy cells, is able to totally block DNA fragmentation in apoptotic cells indicating that DFF45 is the main inhibitor of apoptotic DNAse activity in non apoptotic cells. Finally, the key role of DFF45/DFF40 in DNA fragmentation during apoptosis has been demonstrated *in vivo* using DFF45 knock-out mice where DNA fragmentation activity was totally abolished in cells from DFF45 mutant tissues, whereas normal immune system development occurs (Zhang J. Proc. Natl. Acad. Sci. USA 95: 12480-85, 1998). Altogether these data suggest that DFF is critical for DNA fragmentation, a crucial event in apoptosis.

Recently a novel family of apoptosis effectors sharing homology with DFF45 has been identified by Inohara *et al.* (EMBO J. 17:2526-33, 1998) and called Cell death Inducing DFF45-like Effector (CIDE). CIDE A was identified in mouse and human whereas CIDE B was only identified in mouse. Unlike DFF45, these proteins, when transfected into cells induce apoptosis, membrane blebbing and DNA fragmentation by an unknown mechanism which is inhibited by DFF45 but not by caspase inhibitors. Mutant analysis indicates that the N terminal domain of CIDE is involved in the inhibition by DFF45 whereas the C-terminal domain of CIDE is necessary to induce apoptosis. Homologous domains to N-terminal CIDE domains were also identified in FSP27 (Fat Specific Protein, a murine protein of unknown function) and DREP1, a *Drosophila melanogaster* homolog to DFF45. A similar regulatory domain has been identified by mutational analysis in the N-terminal part of DFF40 and it may regulate endonuclease activity of the catalytic domain (C-terminal part) by homophilic interaction with DFF45 (Inohara N. *et al,* J. Biol. Chem. 274, 270-4, 1999).

This indicates that CIDE family proteins have an established, proven history as therapeutic targets and that they can play a role in preventing, ameliorating or correcting dysfunctions or diseases, including but not limited to cancer, hematological disorders, e.g. bone marrow failure including myelodysplastic syndromes, aplastic anemia, neutropenia and leukemia, cardiovascular diseases, liver diseases, neurodegenerative pathologies.

The invention relates to a new member of the CIDE family, HLYAL47, similar to other family members such as CIDE B, in controlling programmed cell death and inducing DNA fragmentation during apoptosis.

In accordance with a first aspect of the invention, there are provided HLYAL47 polynucleotides and polypeptides, which are CIDE family proteins, as well as recombinant materials and methods for their production.

Another aspect of the invention relates to methods for using such HLYAL47 polypeptides and polynucleotides, for example in the treatment of cancer, hematological disorders, e.g. bone marrow failure including myelodysplastic syndromes, aplastic anemia, neutropenia and leukemia, cardiovascular diseases, liver diseases, neurodegenerative pathologies.

In still another aspect, the invention relates to methods to identify agonists and antagonists of HLYAL47 using the materials provided by the invention, and treating conditions associated with expression of HLYAL47.

Yet another aspect of the invention relates to diagnostic assays for detecting diseases associated with inappropriate HLYAL47 activity or levels.

The following drawings are illustrative of the embodiments of the invention and are not meant to limit the scope of the invention as encompassed by the claims.

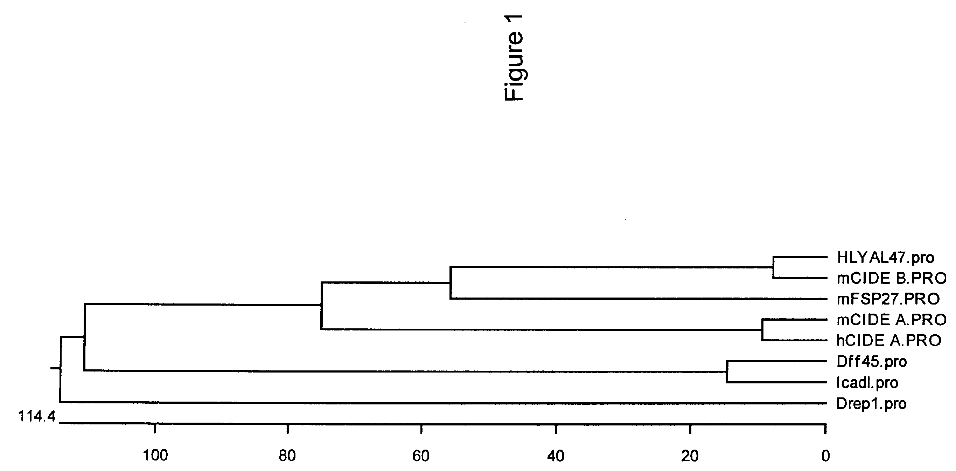

Figure 1 illustrates the phylogenic relationship between HLYAL47 and other members of CIDE protein and DFF45 family. The length of each branch represents the distance between sequence pairs.

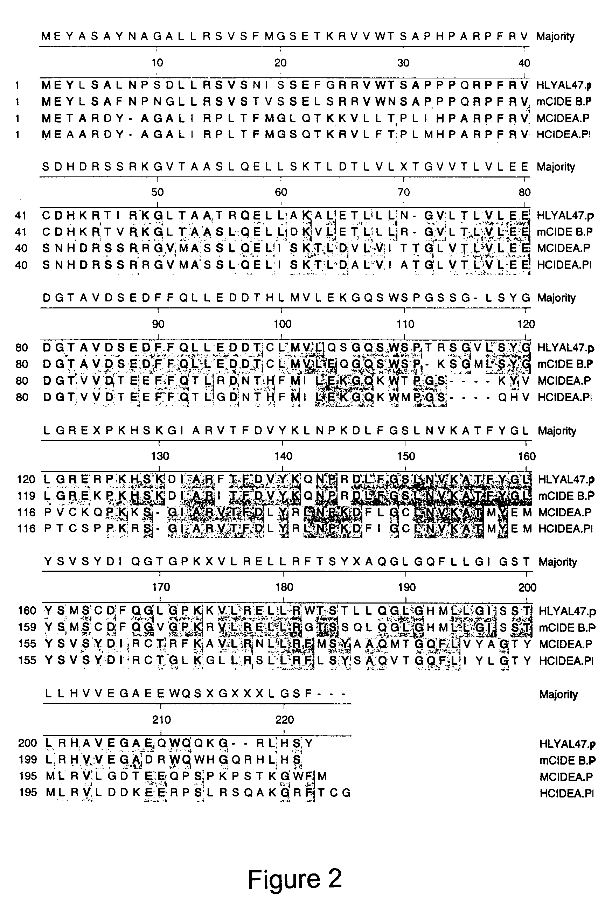

Figure 2 shows an amino acid sequence comparison of HLYAL47 with other members of the CIDE family from the phylogenic tree of Figure 1. The alignment is performed with the clustal algorithm of MEGALIGN (version 3.18) from DNASTAR. Regions of homology to other members of the family are shaded in black.

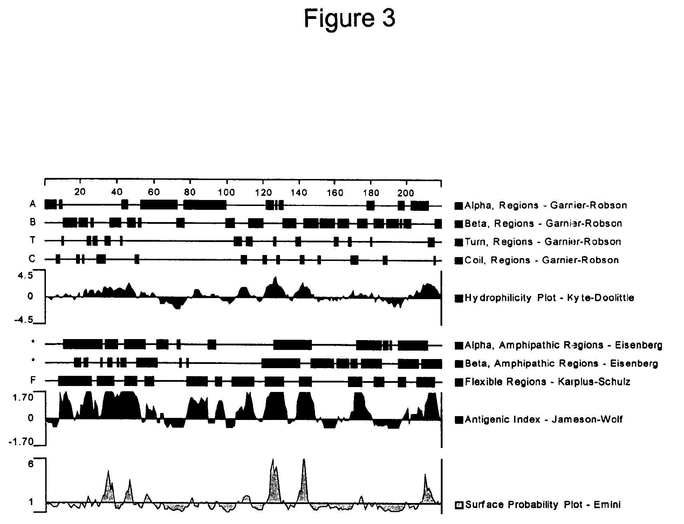

Figure 3 illustrates secondary structural features of HLYAL47 protein with the hydrophilicity, hydrophobicity, the propensity to generate alpha helix, beta sheet, turn or coiled regions, the probability of regions to be on the surface, and the flexible regions. The boxed areas are those which correspond to the regions indicated. The hydrophobicity plot illustrates hydrophobic areas of the protein sequence. The antigenicity of the protein fragments is higher in areas exposed to the surface, which are hydrophilic and flexible regions. The analysis was performed with Protean (version 3.14) from DNASTAR.

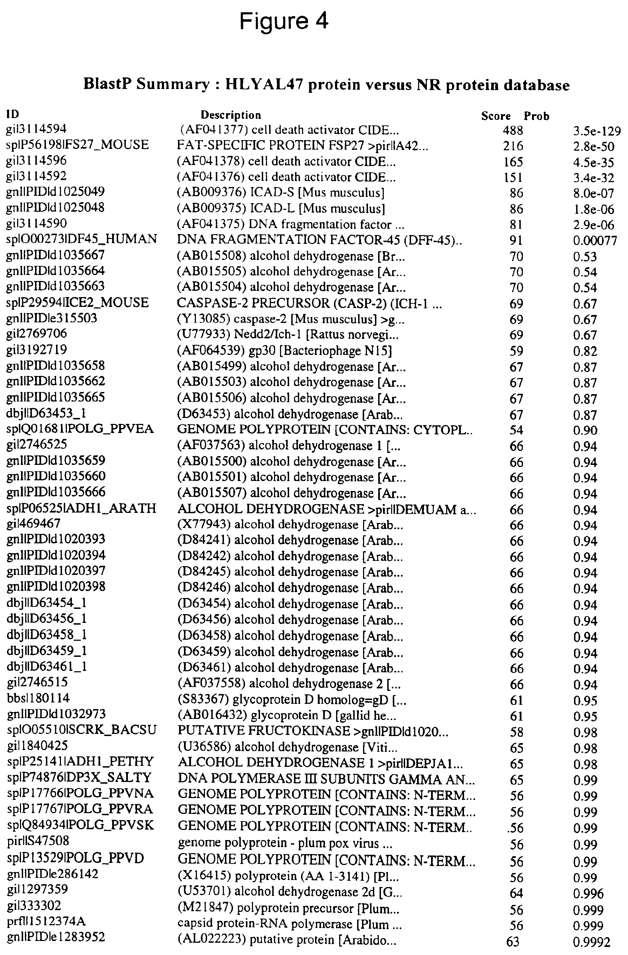

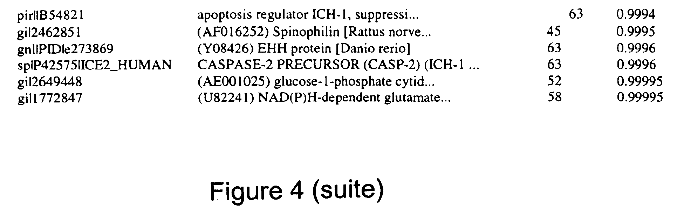

Figure 4 shows a BLAST search against non redundant protein databases with the HLYAL47 sequence as a query, highlighting that HLYAL47 is a new human protein belonging to the CIDE gene family.

Figure 5 shows HLYAL47 mRNA human tissue distribution determined by Northern Blot (from CLONTECH) analysis using a cDNA ³²P-labeled probe.

The following definitions are provided to facilitate understanding of certain terms used frequently herein.

"HLYAL47 activity", "HLYAL47 polypeptide activity" or "biological activity of HLYAL47" refers to the metabolic or physiologic function of said HLYAL47, including similar activities or improved activities or these activities with decreased undesirable side-effects. Also included are antigenic and immunogenic activities of said HLYAL47.

"HLYAL47" or "HLYAL47 polypeptide" refers generally to a polypeptide comprising the amino acid sequence set forth in SEQ ID NO:2 or an allelic variant thereof, as well as variants as described further below.

"HLYAL47 gene" or "HLYAL47 polynucleotide" refer among others to a polynucleotide comprising the nucleotide sequence set forth in SEQ ID NO:1 or allelic variants thereof and/or their complements as described in the following specification.

"Antibodies" as used herein includes polyclonal and monoclonal antibodies, chimeric, single chain, and humanized antibodies (an antibody - mouse or any other - with some specific parts changed to human homologous regions), as well as Fab fragments, including the products of a Fab or other immunoglobulin expression library.

"Isolated" means altered by the hand of man from the natural state. If an "isolated" composition or substance occurs in nature, it has been changed or removed from its original environment, or both. For example, a polynucleotide or a polypeptide naturally present in a living animal is not "isolated" but the same polynucleotide or polypeptide separated from the coexisting materials of its natural state is "isolated", as the term is employed herein. For example, recombinant DNA molecules contained in a vector are considered isolated for the purposes of the present invention. Further examples of isolated nucleic acid molecules include recombinant DNA molecules maintained in heterologous host cells or purified DNA molecules in solution. Isolated RNA molecules include *in vivo* or *in vitro* RNA transcripts of the DNA molecules of the present invention. Isolated nucleic acid molecules according to the invention further include such molecules produced synthetically.

"Polynucleotide" generally refers to any polyribonucleotide or polydeoxyribonucleotide, which may be unmodified RNA or DNA or modified RNA or DNA. "Polynucleotide" includes, without limitation single-and double-stranded DNA, DNA that is a mixture of single-and double-stranded regions, single-and double-stranded RNA, and RNA that is a mixture of single-and double-stranded regions, hybrid molecules comprising DNA and RNA that may be single-stranded or, more typically, double-stranded or a mixture of single-and double-stranded regions. In addition, "polynucleotide" refers to triple-stranded regions comprising RNA or DNA or both RNA and DNA. The term polynucleotide also includes DNA or RNA containing one or more modified bases and DNA or RNA with backbones modified for stability or for other reasons. "Modified" bases include, for example, tritylated bases and unusual bases such as inosine. A variety of modifications have been made to DNA and RNA; thus, "polynucleotide" embraces chemically, enzymatically or metabolically modified forms of polynucleotides as typically found in nature, as well as the chemical forms of DNA and RNA characteristic of viruses and cells. Thus, "polynucleotide" also encompasses variants of the nucleic acid molecules of the invention which encode portions, analogs or derivatives of the HLYAL47 polypeptides. Variants may occur naturally, such as natural allelic variant. By an "allelic variant" is intended one of several alternate forms of a gene occupying a given locus on a chromosome of an organism. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins-structure and molecular properties, 2nd Ed., T. E. Creighton, W. H. Freeman and Company, New York, 1993 and Wold, F., Post-translational Protein Modifications: Perspectives and Prospects, pp. 1-12 in Post-translational covalent modification of proteins, B. C. Johnson, Ed., Academic Press, New York, 1983; Seifter *et al.*,"Analysis for protein modifications and nonprotein cofactors", *Meth Enzymol* (1990) 182:626-646 and Rattan *et al.,* "Protein Synthesis: Post-translational Modifications and Aging", *Ann NY Acad Sci* (1992) 663:48-62.

"Variant" as the term is used herein, represents a polynucleotide or polypeptide that differs from a reference polynucleotide or polypeptide respectively, but retains essential properties. A typical variant of a polynucleotide differs in nucleotide sequence from a reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g. : (Computational Molecular Biology, Lesk, A. M., ed., Oxford University Press, New York, 1988; Biocomputing Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exists a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J. Molec. Biol.* (1990) 215:403).

As an illustration, by a polynucleotide having a nucleotide sequence having at least, for example, 95% "identity" to a reference nucleotide sequence is intended a polynucleotide in which the nucleotide sequence is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended a polypeptide having an amino acid sequence identical to the reference sequence except that the polypeptide sequence may include up to five amino acid alterations per each 100 amino acids of the reference amino acid sequence. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

In one aspect, the present invention relates to HLYAL47 polypeptides. The HLYAL47 polypeptides include the polypeptide of SEQ ID NO:2; as well as polypeptides comprising the amino acid sequence of SEQ ID NO:2; and polypeptides comprising the amino acid sequences which present at least 80% identity to that of SEQ ID NO:2 over its entire length, and still more preferably at least 90% identity, and even still more preferably at least 95% identity to SEQ ID NO:2. Furthermore, those with at least 97-99% are highly preferred. Also included within HLYAL47 polypeptides are polypeptides having an amino acid sequence which has at least 80% identity to the polypeptide having the amino acid sequence of SEQ ID NO:2 over its entire length, and still more preferably at least 90% identity, and even still more preferably at least 95% identity to SEQ ID NO:2. Furthermore, those with at least 97-99% are highly preferred. HLYAL47 polypeptides comprising an amino acid sequence having at least 80% identity, preferably at least 90% identity, and even still more preferably at least 95% to the amino acid sequence encoded by the cDNA contained in pcDNA3 / HLYAL47 as discussed below are also included within the present invention.

It will be recognized in the art that some amino acids of the HLYAL47 polypeptides can be varied without significant effect on the structure or function of the protein. Guidance concerning with amino acid changes which are likely to be phenotypically silent can be found in Bowie, J. U. et al, "Deciphering the Message in Protein Sequences : Tolerance to Amino Acid Substitutions", *Science* 247 : 1306-1310 (1990).

Thus, the invention further provides variants of the HLYAL47 polypeptides which show substantial HLYAL47 polypeptide activity or which include regions of HLYAL47 polypeptide such as the protein portions discussed below. Such mutants include HLYAL47 polypeptides in which deletions, insertions, type substitutions, inversions and/or repeats have occured.

Thus, the fragment, derivative or analog of the polypeptide set forth in SEQ ID NO:2, or that encoded by the deposited cDNA as discussed below, may be :
- one in which one or more of the amino acid residues are substituted with a conserved or non-conserved amino acid residue, preferably a conserved amino acid residue, and such substituted amino acid residue may or may not be one encoded by the genetic code, or
- one in which one or more of the amino acid residues includes a substituent group, or
- one in which the mature polypeptide is fused with another compound, such as a compound for increasing the half-life of the polypeptide (for example, polyethylene glycol), or
- one in which additional amino acids are fused to the mature polypeptide, such as the IgG Fc fusion region peptide or leader or secretory sequence which is employed for purification of the mature polypeptide or a pro-protein sequence. Such fragments, derivatives and analogs are deemed to be within the scope of those skilled in the art.

Of particular interest are substitutions of charged amino acids with another charged amino acid and with neutral or negatively charged amino acids. The latter results in proteins with reduced positive charge to improve the characteristics of the HLYAL47 polypeptide, for example for prevention of aggregation.

As indicated, changes are preferably of a minor nature, such as conservative amino acid substitutions that do not significantly affect the activity of the protein.

Examples of amino acids which can be used for substitution are given hereafter.
- Aromatic: Phenylalanine
Tryptophan
Tyrosine
- Hydrophobic: Leucine
Isoleucine
Valine
- Polar: Glutamine
Asparagine
- Basic: Arginine
Lysine
Histidine
- Acidic: Aspartic acid
Glutamic acid
- Small: Alanine
Serine
Threonine
Methionine
Glycine

Generally, the number of amino acid substitutions will not be more than 50.

Amino acids in the HLYAL47 polypeptide of the present invention that are essential for function can be identified by methods known in the art, such as site-directed mutagenesis or alanine-scanning mutagenesis (Cunnigham and Wells, Science 244 : 1081-1085 (1989)). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis, e.g. crystallization, nuclear magnetic resonance or photoaffinity labeling (Smith et al., J. *Mol. Biol.* 224 : 899-904 (1992); de Vos et al., Science 255 : 306-312 (1992)).

As to the selection of peptides or polypeptides bearing an antigenic epitope, it is well known that relatively short synthetic sequences that mimic part of a protein sequence are routinely capable of eliciting an antiserum that reacts with the partially mimicked protein. See for instance Sutcliffe, J. G., Shinnick, T. M. , Green, N. and Learner, R. A., Antibodies that React with Predetermined Sites on Proteins, *Science* 219 : 660-666 (1983). Peptides capable of eliciting protein-reactive sera are frequently represented in the primary sequence of a protein, can be characterized by a set of simple chemical rules, and are confined neither to immunodominant regions of intact proteins (i.e. immunogenic epitopes) nor to the amino or carboxyl terminals.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the invention preferably contain a sequence of at least seven, more preferably at least nine and most preferably between about 15 to about 30 amino acids contained within the amino acid sequence of a polypeptide according to the invention.

For instance, antigenic polypeptides or peptides that can be used to generate HLYAL47 specific antibodies comprise : a polypeptide comprising amino acid residues from about 12 to about 26 of SEQ ID NO:2, a polypeptide comprising amino acid residues from about 31 to about 52 of SEQ ID NO:2, a polypeptide comprising amino acid residues from about 77 to about 89 of SEQ ID NO:2, a polypeptide comprising amino acid residues from about 104 to about 115 of SEQ ID NO:2, a polypeptide comprising amino acid residues from about 120 to about 133 of SEQ ID NO:2, a polypeptide comprising amino acid residues from about 137 to about 149 of SEQ ID NO:2, a polypeptide comprising amino acid residues from about 168 to about 180 of SEQ ID NO:2.

The epitope-bearing peptides and polypeptides of the invention can be prepared by any conventional means, for instance by the method described in document US patent n° 4,631,211.

HLYAL47 polypeptides of the present invention and the epitope-bearing fragments thereof can be combined with parts of the constant domain of immunoglobulins (IgG), resulting in chimeric polypeptides. These fusion proteins facilitate the purification and generally show an increased half life *in vivo.* See for instance Traunecker et al., *Nature* 331 : 84-86 (1988). Fusion proteins that have a disulfide-linked dimeric structure due to the IgG part can also be more efficient in binding and neutralizing other molecules than the monomeric HLYAL47 protein or protein fragment thereof (Fountoulakis et al., J. *Biochem.* 270 : 3958-3964 (1995)).

The HLYAL47 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of HLYAL47 polypeptides or variants, mutated forms, derivatives thereof as hereabove mentioned are also included in the invention. A fragment is a polypeptide having an amino acid sequence that entirely is the same as part, but not all, of the amino acid sequence of the aforementioned HLYAL47 polypeptides. As with HLYAL47 polypeptides, fragments may be "free-standing" or comprised within a larger polypeptide of which they form a part or region, most preferably as a single continuous region.

Preferred fragments include, for example, truncation polypeptides having the amino acid sequence of HLYAL47 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate protein activity, including those with a similar activity or an improved activity, or with a decreased undesirable activity. Also included are those that are antigenic or immunogenic in an animal, especially in a human.

Preferably, all of these polypeptide fragments retain the biological activity of the protein, including antigenic activity.

The HLYAL47 polypeptides of the invention can be prepared in any suitable manner. Such polypeptides include isolated naturally occurring polypeptides, recombinantly produced polypeptides, or polypeptides produced by a combination of these methods.

In one embodiment, the HLYAL47 polypeptides of the invention can be prepared by chemical synthesis, for example by using non natural and/or modified amino acids. Thus, it can be advantageous to use non natural amino acids, for example D-amino acids, to enhance life time of the polypeptides, or analogs of amino acids, for example sulfurized forms of amino acids.

Methods for preparing such polypeptides are known in the art.

For example, chemical synthesis of the polypeptides of the invention can be carried out by using a solid phase process. In such a method, the C-terminal moiety of an amino acid is protected by using conventional protective groups (e.g. BOC or FMOC) and the thus protected amino acid is fixed onto an inert solid support by its C-terminal region. At the end of this step, the protective group is removed and a second amino acid, optionally protected in a similar manner, is fixed. The N-terminal protective groups are removed after each amino acid is fixed, protective groups possibly present on the lateral chains being conserved. When the peptide chain is synthetized, the peptide is cleaved from the support and the remaining protective groups are removed. Coupling can be carried out by using N,N'-diisopropyl-carbodiimine (DIC), among others, in a solvent of DMF or DCM.

This solid phase synthesis is described in for example Stewart J. M. et al., Solid Phase Peptides Synthesis. Pierce Chem. Company, Rockford, 111, 2^{nd} Ed., (1984).

Other polypeptides according to the invention comprise the amino acid sequence of the HLYAL47 polynucleotide which possesses the complete amino acid sequence encoded by the cDNA clone contained in ATCC deposit number PTA-188 as discussed below, as well as polypeptides which comprise the amino acid sequence of the mature HLYAL47 polynucleotide which possesses the complete amino sequence encoded by the cDNA clone contained in ATCC deposit number PTA-188.

In another aspect, the invention provides a peptide or polypeptide comprising an epitope-bearing portion of a polypeptide of the invention. The epitope of this polypeptide portion is an immunogenic or antigenic epitope of a polypeptide described in the present invention. An immunogenic epitope is defined as a part of a protein that elicits an antibody response when the whole protein is the immunogen. On the other hand, a region of a protein molecule to which an antibody can bind is defined as an antigenic epitope.

Another aspect of the invention relates to HLYAL47 polynucleotides.

Unless otherwise indicated, all nucleotide sequences determined by sequencing a DNA molecule herein have been determined by using an automated DNA sequencer, and all amino acid sequences of polypeptides encoded by DNA sequences determined herein have been predicted by translation of a DNA sequence determined as above. Therefore, as is known in the art for any DNA sequence determined by this approach, any nucleotide sequence may contain some errors. Nucleotide sequences determined by automation are typically at least about 90 % identical, more typically at least about 95 % to about 99.9 % identical to the actual nucleotide sequence of the sequenced DNA molecule.

HLYAL47 polynucleotides include isolated polynucleotides which encode the HLYAL47 polypeptides and fragments, and polynucleotides closely related thereto, e.g. variants, derivatives, mutated forms. More specifically, HLYAL47 polynucleotides of the invention include a polynucleotide comprising the nucleotide sequence set forth in SEQ ID NO:1 encoding a HLYAL47 polypeptide of SEQ ID NO:2, and a polynucleotide having the particular sequence of SEQ ID NO:1.

HLYAL47 polynucleotides according to the invention further include a polynucleotide comprising a nucleotide sequence that has at least 80% identity to a nucleotide sequence encoding the HLYAL47 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under HLYAL47 polynucleotides are nucleotide sequences which have sufficient identity to a nucleotide sequence contained in SEQ ID NO:1 or contained in the cDNA insert in the plasmid deposited with the ATCC Deposit number PTA-188 as discussed below to hybridize under conditions useable for amplification or for use as a probe or marker. Moreover, HLYAL47 polynucleotides include a nucleotide sequence having at least 80% identity to a nucleotide sequence encoding the HLYAL47 polypeptide expressed by the cDNA insert deposited at the ATCC with Deposit Number PTA-188, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above HLYAL47 polynucleotides.

A deposit containing a HLYAL47 cDNA has been deposited with the American Type Culture Collection (ATCC), 10801 University Blvd, Manassas, VA 20110-2209, USA, on June 08, 1999, and assigned ATCC Deposit Number PTA-188. The deposited material (clone) is a bluescript plasmid (obtainable from Stratagene) which contains the full length HLYAL47 cDNA, referred to as SLCA1 upon deposit. The cDNA insert is within *Sall / Not/* restriction sites in the vector. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

HLYAL47 of the invention is structurally related to other proteins of the CIDE family, as shown by the results of sequencing the cDNA of SEQ ID NO:1 encoding HLYAL47. The cDNA sequence of SEQ ID NO:1 contains an open reading frame which begins in nucleotide position 133 and ends in nucleotide position 792 encoding a polypeptide of 219 amino acids of SEQ ID NO:2 with a putative molecular weight of 24.7 kDa.

Amino acid sequence of SEQ ID NO:2 has about 85.7 % identity (using GAP) in amino acid residues with mouse CIDE B and 41.6% human CIDE A (Inohara *et al.* EMBO J. 17:2526-33, 1998). Nucleotide sequence of SEQ ID NO:1 has about 76 % identity (using GAP) in nucleotide sequence with mouse CIDE B.

The polynucleotides of the invention encoding HLYAL47 may be obtained using standard cloning and screening, from a cDNA library derived from mRNA in cells of human origin using the expressed sequence tag (EST) analysis (Adams, M. D., *et al. Science* (1991) 252:1651-1656; Adams, M. D. *et al., Nature,* (1992) 355:632-634; Adams, M. D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding HLYAL47 polypeptide of SEQ ID NO:2 may be identical to the polypeptide encoding sequence contained in SEQ ID NO:1, or it may be a different sequence, which as a result of the redundancy (degeneracy) of the genetic code, also encodes the polypeptide of SEQ ID NO:2.

As indicated, the invention also provides the mature form of the HLYAL47 polypeptide of the invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species on the protein. Further, it has long been known that the cleavage specificity of a secreted protein is ultimately determined by the primary structure of the complete protein, i.e. its amino acid sequence. Therefore, the invention provides a nucleotide sequence encoding the mature HLYAL47 polypeptide having the amino acid sequence encoded by the cDNA clone contained in the host identified as ATCC Deposit PTA-188. By the mature HLYAL47 polypeptide having the amino acid sequence encoded by the cDNA clone contained in the host identified as ATCC Deposit PTA-188 is meant the mature form of the HLYAL47 polypeptide produced by expression in a mammalian cell of the complete open reading frame encoded by the human DNA sequence of the clone contained in the vector in the deposited host. As indicated, the mature HLYAL47 receptor having the amino acid sequence encoded by the cDNA clone contained in ATCC Deposit PTA-188 may or may not differ from the predicted "mature" HLYAL47 protein set forth in SEQ ID NO:2, depending on the accuracy of the predicted cleavage site based on computer analysis.

Methods for predicting whether a protein has a secretory leader as well as the cleavage site of this leader sequence are available. For instance, the methods of Mc Geoch *(Virus Res.* 3:271-286 (1985)) and von Heinje *(Nucleic Acids Res.* 14:4683-4690 (1986)) can be used.

When the polynucleotides of the invention are used for the recombinant production of HLYAL47 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro-or prepro-protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding HLYAL47 variants comprising the amino acid sequence of HLYAL47 polypeptide of SEQ ID NO:2 in which several, for example 5-10, 1-5, 1-3, 1-2 or 1 amino acid residues are substituted, deleted or added, in any combination.

The invention is further directed to fragments of the isolated nucleic acid molecules described herein. By a fragment of an isolated nucleic acid molecule having the nucleotide sequence of the deposited cDNA or the nucleotide molecule having the nucleotide sequence shown in SEQ ID NO:1 is intended fragments of at least about 15 nucleotides, and preferably of at least about 20 nucleotides, more preferably of at least about 30 nucleotides, even more preferably of at least about 40 nucleotides, which are useful as diagnostic probes and primers. Of course, larger fragments of about 50-1500 nucleotides in length are also useful according to the present invention, as are fragments corresponding to most, if not all, of the nucleotide sequence of the deposited cDNA or as shown in SEQ ID NO:1. By a fragment of at least 20 nucleotides in length, for example, are intended fragments which include 20 or more contiguous bases from the nucleotide sequence of the deposited cDNA or from the sequence shown in SEQ ID NO:1, deleted of a continous series of residues including the 5' end or 3' end or both.

Preferred nucleic acid fragments according to the invention comprise nucleic acid molecules which comprises nucleotide position 133 to nucleotide position 792 of the nucleotide sequence shown in SEQ ID NO:1.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number PTA-188 or a fragment thereof, may be used as hybridization probes for cDNA and genomic DNA, to isolate full-length cDNAs and genomic clones encoding HLYAL47 and to isolate cDNA and genomic clones of other genes that have a high sequence similarity to the HLYAL47 gene. Such hybridization techniques are known to those skilled in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, obtaining a polynucleotide encoding HLYAL47 polypeptide comprises the steps of screening an appropriate library under stringent hybridization conditions with a labeled probe having the SEQ ID NO:1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics for animal and human diseases.

The invention also relates to recombinant vectors which comprise a polynucleotide or polynucleotides according to the invention and to an expression system capable of producing the HLYAL47 polypeptide when said expression system is present in a compatible host cell. The invention also relates to host cells which are genetically engineered with the recombinant vectors of the invention, and to the production of the polypeptides according to the invention by recombinant techniques. Cell-free translation systems can also be employed to produce such proteins using RNAs derived from the DNA constructs of the invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *Basic Methods in Molecular Biology* (1986) and Sambrook et al., *Molecular Cloning : a Laboratory Manual,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

The DNA insert should be operatively linked to an appropriate promoter, such as the bacterial phage lambda PL and RP, T3 and T7 promoters, the *E. coli lac, trp* and *tac* promoters, the eukaryotic SV40 early and late promoters, CMV immediate early promoters and eukaryotic promoters of retroviral LTRs, such as those of the Rous Sarcoma Virus (RSV), to name a few. Other suitable promoters will be known to the skilled artisan.

The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

The expression vectors will preferably include at least one selectable marker. Such markers include dihydrofolate reductase, zeocin, hygromycin, or neomycin resistance for eukaryotic cell culture and tetracycline or ampicillin resistance genes for culturing in *E. coli* and other bacteria.

Representative examples of appropriate hosts include, but are not limited to, bacterial cells, such as streptococci, staphylococci, *E. coli, Bacillus subtilis, Streptomyces* and *Salmonella typhimurium* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells. Other examples of useable hosts are given hereafter. Appropriate culture mediums and conditions for the above-described host cells are known in the art.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. Generally, any system or vector suitable to maintain, propagate or express polynucleotides to produce a polypeptide in a host may be used.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

The appropriate nucleotide sequence may be inserted into an expression vector by any of a variety of well-known and routine techniques, such as, for example, those set forth in Sambrook et al, Molecular Cloning, a laboratory manual (supra).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the HLYAL47 polypeptide is to be expressed for use in screening assays, generally, it is preferred that the polypeptide be produced at the surface of the cell. In this event, the cells may be harvested prior to use in the screening assay. If HLYAL47 polypeptide is secreted into the medium, the medium can be recovered in order to recover and purify the polypeptide; if produced intracellularly, the cells must first be lysed before the polypeptide is recovered.

The polypeptides of the invention may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 discloses fusion proteins comprising various portions of constant region of immunoglobulin molecules together with another human protein or part thereof. In many cases, the Fc part in a fusion protein is advantageous for use in therapy and diagnosis. On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as, hlL5- has been fused with Fc portions for the purpose of high-throughput screening assays to identify antagonists of hlL-5. See, D. Bennett *et al., Journal of Molecular Recognition, Vol. 8:*52-58 (1995) and K. Johanson *et al., The Journal of Biological Chemistry, Vol. 270,* No.16:9459-9471 (1995).

HLYAL47 protein can be recovered and purified from recombinant cell cultures by well-known methods, including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

It is deemed that mammals with certain diseases or susceptibility to certain diseases express significantly altered, enhanced or diminished levels of HLYAL47 protein and mRNA encoding HLYAL47, when compared to a corresponding "standard" mammal, i.e. a mammal of the same species not having or not presenting a susceptibility to the disease. Further, it is deemed that significantly altered, enhanced or diminished levels of HLYAL47 protein can be detected in certain body fluids (e.g. bone marrow, lymph fluid, blood, sera, plasma, saliva, urine, synovial fluid and spinal fluid) from mammals with the disease or presenting a susceptibility to the disease when compared to body fluids from mammals of the same species not having or not presenting a susceptibility to the disease. Thus, the invention provides a diagnostic method which involves assaying the expression level of the gene encoding a HLYAL47 polypeptide of the invention in mammalian cells or body fluid and comparing the gene expression level with a standard HLYAL47 expression level, whereby an altered, enhanced or diminished expression level of the gene with respect to the standard is indicative of the disease or of a susceptibility to the disease. Preferred mammals include monkeys, apes, cats, dogs, cows, pigs, horses, rabbits and humans.

Diseases which can be diagnosed include but are not limited to cancer, hematological disorders, e.g. bone marrow failure including myelodysplastic syndromes, aplastic anemia, neutropenia and leukemia, cardiovascular diseases, liver diseases, neurodegenerative pathologies.

By "assaying the expression level of the gene encoding a HLYAL47 polypeptide" is intended qualitatively or quantitatively measuring or estimating the level of a HLYAL47 protein or the level of the mRNA encoding a HLYAL47 polypeptide in a first biological sample either directly (e.g. by determining or estimating absolute protein level or mRNA) or relatively (e.g. by comparing to the HLYAL47 protein level or mRNA in a second biological sample).

Preferably, the HLYAL47 protein level or mRNA level in the first biological sample is measured or estimated and compared to a standard HLYAL47 protein level or mRNA level, the standard being taken from a second biological sample obtained from an individual not having (nor presenting a susceptibility to) the disease. As will be appreciated , once a standard HLYAL47 protein level or mRNA level is known, it can be used repeatedly as a standard for comparison.

By "biological sample" is intended any biological sample, either tissue sample or body fluid as above indicated, obtained from an individual, cell line, tissue culture, or other source which may contain HLYAL47 protein or mRNA.

Total cellular RNA can be isolated from a biological sample using the single-step guanidinium-thiocyanate-phenol-chloroform method described in Chomczynski and Sacchi, *Anal. Biochem.* 162 : 156-159 (1987). Levels of mRNA encoding the HLYAL47 polypeptide are then assayed using any appropriate method. These include Northern Blot analysis (Harada et al., *Cell* 63 : 303-312 (1990)), S1 nuclease mapping (Fujita et al., *Cell* 49 : 357-367 (1987)), the polymerase chain reaction (PCR), reverse transcription in combination with the polymerase chain reaction (RT-PCR) (Fujita et al., *Cell* 49 : 357-367 (1987)), and reverse transcription in combination with the ligase chain reaction (RT-LCR).

Assaying HLYAL47 protein levels in a biological sample can occur using antibody-based techniques. For example, HLYAL47 protein expression can be studied with classical immunohistological methods (Jalkanen, M., et al., *J. Cell. Biol.* 101 : 976-985 (1985); Jalkanen, M., et al., *J. Cell. Biol. 105 : 3087-3096 (1987)).* Other antibody-based methods useful for detecting HLYAL47 gene expression include immunoassays, such as the enzyme linked immunosorbent assay (ELISA) and the radioimmunoassay (RIA).

Suitable labels are known in the art and include enzyme labels, such as glucose oxidase, and radioisotopes, such as iodine (¹²⁵l, ¹²¹l), carbon (¹⁴C), sulfur (³⁵S), tritium (³H), indium (^{l12}ln), and technetium (^{99m}Tc), and fluorescent labels, such as fluorescein and rhodamine and biotin.

In addition, the invention provides a diagnostic assays for diagnosing or determining a susceptibility to, among others, cancer, hematological disorders, e.g. bone marrow failure including myelodysplastic syndromes, aplastic anemia, neutropenia and leukemia, cardiovascular diseases, liver diseases, neurodegenerative pathologies, through detection of mutation in the HLYAL47 gene by the methods described below.

Detection of a mutated form of HLYAL47 gene associated with a dysfunction will provide a diagnostic tool that can add to or define a diagnosis of a disease or susceptibility to a disease which results from under-expression, over-expression or altered expression of HLYAL47. Individuals carrying mutations in the HLYAL47 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HLYAL47 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton et *al., Proc. Natl. Acad. Sci. USA* (1985) 85: 4397-4401. In another embodiment, an array of oligonucleotides probes comprising HLYAL47 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The nucleic acid molecules of the present invention are also valuable for chromosome identification. The sequence is specifically targeted to and can hybridize with a particular location on an individual human chromosome. The mapping of DNAs to chromosomes according to the invention is an important first step in correlating those sequences with genes associated with disease. Once a sequence has been mapped to a precise chromosomal location, the physical position of the sequence on the chromosome can be correlated with genetic map data. Such data are found, for example, in V. McKusick, Mendelian Inheritance In Man, available on-line through Johns Hopkins University, Welch Medical Library. The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The polypeptides of the invention or their fragments, derivatives or analogs thereof as described above, or cells expressing them can also be used as immunogens to produce monoclonal or polyclonal antibodies immunospecific for HLYAL47 polypeptides. The term "immunospecific" means that the antibodies have substantially greater affinity for the polypeptides of the invention than their affinity for other related polypeptides.

Antibodies generated against the HLYAL47 polypeptides can be obtained by administering the polypeptides or epitope-bearing fragments, analogs or cells to an animal, preferably a non human, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor et al., Immunology Today (1983) 4:72) and the EBV-hybridoma technique (Cole et al., Monoclonal Antibodies and Cancer Therapy, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies. Non-limiting examples of polypeptides or peptides that can be used to generate HLYAL47 polypeptide-specific antibodies include : a polypeptide comprising amino acid residues from about 19 to about 26 of SEQ ID NO:2 ; a polypeptide comprising amino acid residues from about 31 to about 52 of SEQ ID NO:2; a polypeptide comprising amino acid residues from about 77 to about 89 of SEQ ID NO:2; a polypeptide comprising amino acid residues from about 104 to about 115 of SEQ ID NO:2 ; a polypeptide comprising amino acid residues from about 120 to about 133 of SEQ ID NO:2; a polypeptide comprising amino acid residues from about 137 to about 149 of SEQ ID NO:2; a polypeptide comprising amino acid residues from about 168 to about 180 of SEQ ID NO:2. As indicated above, the inventors have determined that the above polypeptide fragments correspond to antigenic regions of HLYAL47 polypeptide.

The above mentioned antibodies may be employed to isolate or to identify clones expressing the polypeptide or to purify the polypeptides by affinity chromatography.

Antibodies against HLYAL47 polypeptides may also be employed to treat, among others, patients with myelodysplastic or hepatic disorders, stroke, cardiac diseases or to treat any disorder related to abnormal expression of said HLYAL47 polypeptide.

Another aspect of the invention relates to a method for inducing an immunological response in a mammal which comprises inoculating the mammal with a HLYAL47 polypeptide, or a fragment thereof, adequate to produce antibody and/or T cell immune response to protect said mammal from, among others, renal disorders, hepatic disorders, hypertensive disease, stroke, cardiac diseases or to treat any disorder related to abnormal expression of said HLYAL47 polypeptide.

Yet another aspect of the invention relates to a method of inducing immunological response in a mammal which comprises, delivering to the mammal a HLYAL47 polypeptide, e.g. via a vector directing expression of the HLYAL47 polynucleotide, *in vivo* in order such an immunological response induces the production of antibody for protecting said mammal from diseases.

A further aspect of the invention relates to an immunological/vaccine composition which, when introduced into a mammalian host, induces an immunological response in that mammal to a HLYAL47 polypeptide, wherein said composition comprises a HLYAL47 polypeptide or a HLYAL47 gene as defined hereabove.

The vaccine formulation may further comprise a suitable carrier. Since HLYAL47 polypeptide may be broken down in the stomach, it is preferably administered parenterally (including subcutaneous, intramuscular, intravenous or intradermal injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

HLYAL47 polypeptides of the present invention may be employed in a screening process for candidate compounds which, directly or indirectly, activate (agonists) or inhibit activation of (antagonists, or otherwise called inhibitors) the HLYAL47 polypeptide of the invention. Thus, the polypeptides of the invention may also be used to identify agonists or antagonists from, for example, cells, cell-free preparations, chemical libraries, and natural product mixtures. These agonists and antagonists may be natural substrates, ligands, receptors, etc, as the case may be, of the polypeptide of the invention; or may be structural or functional mimetics of the polypeptide of the invention. See Coligan *et al., Current Protocols in Immunology* 1(2): Chapter 5 (1991).

The choice of the candidate compounds may be based on similarity with known agonists or antagonists of the CIDE family proteins. Similarity of the candidate compounds with compounds known to activate or inhibit activation of CIDE family proteins may be structural or functional, or both. For example, candidate compounds to be screened may be chosen starting from their chemical resemblance with known compounds. Other candidate compounds may be chosen by taking into account their functional relationship with the CIDE family proteins. Other candidate compounds may be chosen among compounds known to be involved in a large number of biological processes and can be selected for screening solely on this basis.

HLYAL47 polypeptides are deemed to be responsible for many biological functions, including many pathologies relating to an abnormal expression of HLYAL47. Accordingly, it is desirous to find compounds and drugs which stimulate HLYAL47 polypeptide activity or its expression (agonists) on the one hand or which can inhibit HLYAL47 polypeptide activity or its expression (antagonists, inhibitors) on the other hand for therapeutic and prophylactic purposes for such conditions as, among others, renal disorders, hepatic disorders, hypertensive disease, stroke, cardiac diseases, or to treat any disorder related to abnormal expression of said HLYAL47 polypeptide.

In general, such screening procedures involve producing appropriate cells which express the protein polypeptide of the present invention on the surface thereof. Such cells include cells from mammals, yeast, *Drosophila* or *E. coli.* Cells expressing the protein (or cell membrane containing the expressed protein) are then contacted with a test compound to observe transport of substances binding, or stimulation or inhibition of a functional response.

The assays may simply test binding of a candidate compound, wherein adherence to the cells bearing the HLYAL47 protein is detected by means of a label directly or indirectly associated with the candidate compound or in an assay involving competition with a labeled competitor. Further, these assays may test whether the candidate compound results in a signal generated by activation of the HLYAL47 protein, using detection systems appropriate to the cells bearing the protein at their surfaces.

Inhibitors of activation are generally assayed in the presence of a known agonist and the effect on activation by the agonist in the presence of the candidate compound is observed. Standard methods for conducting such screening assays are well understood in the art.

Examples of potential HLYAL47 antagonists include antibodies or, in some cases, oligonucleotides or proteins which are closely related to the ligands, substrates, receptors, etc, of the HLYAL47, e.g., a fragment of the ligands, susbtrates, receptors, etc or small molecules which bind to the polypeptide but do not elicit a response, so that the activity of the polypeptide is prevented.

Thus the invention also encompasses agonists or antagonists of HLYAL47 polypeptide identifiable by the methods above described.

The invention also provides methods and compositions for treating abnormal conditions related to both an over-activity or an insufficient activity of HLYAL47.

In a first aspect, the invention provides a method of treating an individual in need of an altered, diminished or inhibited level of HLYAL47 activity or expression, comprising administering to such an individual a pharmaceutical composition comprising an effective amount of an active ingredient capable of altering or diminishing HLYAL47 activity.

In that case, several approaches are available. One approach comprises administering as active ingredient an inhibitor compound (antagonist) identifiable by the method as above described along with a pharmaceutically acceptable carrier, in an amount effective to inhibit activation by blocking activity of HLYAL47, or by inhibiting a second signal, and thereby alleviating the abnormal condition.

In another approach, soluble forms of HLYAL47 polypeptides still capable of binding the ligand, receptor or substrate in competition with endogenous HLYAL47 may be administered as active ingredient. Typical embodiments of such competitors comprise fragments of the HLYAL47 polypeptide.

In still another approach, expression of the gene encoding endogenous HLYAL47 polypeptide can be inhibited using expression blocking techniques. Known such techniques involve the use of anti-sense sequences, either internally generated or separately administered. See, for example, O'Connor, *J Neurochem* (1991) 56:560 in Oligodeoxynucleotides as Anti-sense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

In another aspect, the invention provides a method of treating an individual in need of an increased level of HLYAL47 activity or expression, comprising administering to such an individual a pharmaceutical composition comprising an effective amount of an active ingredient capable of increasing HLYAL47 activity.

For treating abnormal conditions related to an under-expression of HLYAL47 and its activity, several approaches are also available. One approach comprises administering as active ingredient a therapeutically effective amount of a compound which activates HLYAL47, i.e., an agonist identifiable by the method as described above, and/or a therapeutically effective amount of a HLYAL47 polypeptide according to the invention, in combination with a pharmaceutically acceptable carrier, to thereby alleviate the abnormal condition.

Alternatively, gene therapy may be employed to effect the endogenous production of HLYAL47 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996).

In another aspect, the invention provides pharmaceutical compositions for preventing and/or treating in a subject a disease or susceptibility to a disease related to over- or under-activity of HLYAL47.

Pharmaceutical compositions of the invention are intended, but not limited to, the treatment and/or prevention of cancer, hematological disorders, e.g. bone marrow failure including myelodysplastic syndromes, aplastic anemia, neutropenia and leukemia, cardiovascular diseases, liver diseases, neurodegenerative pathologies, among others.

In a first aspect, the pharmaceutical compositions of the invention for preventing and/or treating in a subject a disease or susceptibility to a disease related to over-activity or over-expression of HLYAL47 may comprise a therapeutically effective amount of at least an antagonist of HLYAL47 or a derivative or homologue thereof as defined above, and/or at least a nucleic acid molecule that inhibits the expression of the nucleotide sequence encoding HLYAL47 polypeptide (anti-sense sequence) and/or at least a peptide which competes with HLYAL47 polypeptide for its ligand, receptor or substrate, as discussed above.

In a second aspect, the pharmaceutical compositions of the invention for preventing and/or treating in a subject a disease or susceptibility to a disease related to under-activity or under-expression of HLYAL47 may comprise a therapeutically effective amount of at least an agonist of HLYAL47 or a derivative or homologue thereof as defined above, and/or at least a HLYAL47 polypeptide according to the invention and/or at least a nucleic acid molecule which comprises a nucleotide sequence according to the invention able to express *in vivo* a HLYAL47 polypeptide of the invention.

The pharmaceutical compositions in which at least a polypeptide according to the invention is used may comprise the recombinant vectors of the invention, able to express *in vivo* a polypeptide according to the invention. Polypeptides according to the invention may be also used "naked", i.e. without being inserted into a vector. It has been shown in the art that some DNA or RNA sequences can be expressed in some tissues without the aid of an expression vector.

Pharmaceutical compositions according to the invention, for example those which comprise polypeptides of the invention, such as the soluble form of HLYAL47 polypeptides or small molecules agonists or antagonists, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, intrasternal, intraarterial or intraperitoneal, can be used. Alternative means for systemic administration include transmucosal and transdermal administration using penetrants such as bile salts or fusidic acids or other detergents. In addition, if properly formulated in enteric or encapsulated formulations, oral, rectal, intravaginal administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of about 1µg/kg/day to about 10 mg/kg/day. More preferably, this dose is at least between about 0.01 mg/kg/day and about 1 mg/kg/day. Wide variations in the needed dosage, however, are to be expected in view of the nature of the compounds used and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

The examples below are carried out using standard techniques, which are well known and routine to those of skill in the art, except where otherwise described in detail. The examples illustrate, but do not limit the invention.

### Examples

### Example 1 : Cloning of HLYAL47

The sequence of HLYAL47 was first identified by searching a private database containing approximately 1.5 million human ESTs, which was generated using high throughput automated DNA sequence analysis of randomly selected human cDNA clones (Adams, M.D. *et al., Nature* 377:3-174 (1995); Adams, M.D. *et al., Nature* 355:632-634 (1992); and Adams, M.D. *et al., Science* 252:1651-1656 (1991)). Sequence homology comparisons of each EST were performed against the GenBank database using the blastn and tBlastn algorithms (Altschul, S.F. *et al., J. Mol. Biol.* 215:403-410 (1990)). A specific homology search using the known mouse CIDE B amino acid sequence against this human EST database revealed one EST, from a human spleen chronic lymphocytic leukemia cDNA library, with approximatively 65% similarity to mouse CIDE B cDNA. HLYAL47 contains an insert of 1261 bp and the sequence comparison suggests that it contains the entire HLYAL47 open reading frame coding for a new protein similar to mouse CIDE B.

An expression vector construct was made by inserting the *Eco*RI/*Xh*ol fragment of pKS / HLYAL47, carrying the entire HLYAL47 coding region, into the *Eco*RI/*Xh*ol sites of the expression vector pcDNA3 (Invitrogen, Inc). This construct was named pcDNA3 / HLYAL47. Sequence of the cDNA was confirmed by double strand DNA sequencing and the gene was shown to be completely new by a blast search against Genbank release 107.

### Example 2: Cloning and expression of HLYAL47 in mammalian cells.

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the invention include, for example, vectors such as PSVL and PMSG (Pharmacia, Uppsala, Sweden), pRSVcat (ATCC 37152), pSV2dhfr (ATCC 37146), pBC12Ml (ATCC 67109) and pcDNA3(-) (Invitrogen). Mammalian host cells that could be used include, human Hela 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem. J. 227*:277-279 (1991); Bebbington *et al., Bio/Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pcDNA3(-) contains the strong promoter (CMV) of the Cytomegalovirus. Multiple cloning sites, e.g., with the restriction enzyme cleavage sites *Kpnl, Notl,* facilitate the cloning of the gene of interest. The vectors contain in addition the 3' intron, the polyadenylation and termination signal of the bovine growth hormone gene.

### Example 2(a): Cloning and expression in COS cells.

The expression plasmid, pcDNA3 / HLYAL47 is made by cloning a cDNA encoding HLYAL47 into the expression vector pcDNA3(-) (which can be obtained from Invitrogen, Inc.).

The expression vector pcDNA3(-) contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker; (5) a polyadenylation from the bovine growth hormone gene arranged so that a cDNA can be conveniently placed under expression control of the CMV promoter and operably linked to the polyadenylation signal by means of restriction sites in the polylinker. pcDNA3(-) contains, in addition, the selectable neomycin marker.

A DNA fragment encoding the HLYAL47 polypeptide is cloned into the polylinker region of the vector so that recombinant protein expression is directed by the CMV promoter. The plasmid construction strategy is as follows. The HLYAL47 cDNA is first cloned in the pBluescript vector (Stratagene), it is then excised from the pBluescript vector with *Eco*RI and *Xh*ol. The vector, pcDNA3(-), is digested with *Eco*Ri and *Xh*ol. The digested HLYAL47 cDNA fragment and the linearized vector are then ligated. The ligation mixture is transformed into E. *coli* strain DH5 (available from GIBCO BRL), and the transformed culture is plated on ampicillin media plates which then are incubated to allow growth of ampicillin resistant colonies. Plasmid DNA is isolated from resistant colonies and examined by restriction analysis or other means for the presence of the HLYAL47-encoding fragment.

For expression of recombinant HLYAL47 polypeptide, COS cells are transfected with the above described pcDNA3/HLYAL47 expression vector, using DEAE-Dextran, as described, for instance, in Sambrook *et al., Molecular Cloning: a Laboratory Manual,* Cold Spring Laboratory Press, Cold Spring Harbor, New York (1989). Cells are incubated under conditions for expression of HLYAL47 by the cells.

### Example 2(b): Cloning and expression in HEK293 cells.

The vector pcDNA3/HLYAL47 is used for the expression of HLYAL47 protein. Plasmid pcDNA3/HLYAL47 is described in example 1. The plasmid contains the mouse neomycin resistance gene under control of the SV40 early promoter. HEK293 cells are transfected with this plasmid and can be selected by growing the cells in a selective medium (containing 1 mg/ml Geneticin, Life Technologies). Cells grown in presence of a 1 mg/ml concentration of Geneticin develop resistance to the drug by producing the target enzyme, Neomycin Resistance. If a second gene is linked to the Neomycin Resistance gene, it is usually co-expressed. It is known in the art that this approach may be used to develop cell lines expressing large amounts of the protein of interest, up to 1 pmole per mg of cell membrane in the case of a receptor. Subsequently, when the Geneticin is withdrawn, cell lines are obtained which contain the amplified gene integrated into one or more chromosome(s) of the host cell.

Clontech's Tet-Off and Tet-On gene expression systems and similar systems can be used to express HLYAL47 in a regulated way in mammalian cells (Gossen, M. and Bujard, H., *Proc. Natl. Acad. Sci. USA 89:* 5547-5551(1992)). For the polyadenylation of the mRNA, other signals, e.g., from the human growth hormone or globin genes, can be used as well. Stable cell lines carrying a gene of interest integrated into the chromosomes can also be selected upon co-transfection with a selectable marker such as gpt, G418 or hygromycin.

HEK293 cells are used for transfection. 20 µg of the expression plasmid pcDNA3/HLYAL47 are transfected using calcium phosphate (Chen C, Okayama H, (1987) Mol. Cell. Biol.; 7 : 2745-2752.). The plasmid pcDNA3(-) contains a dominant selectable marker, the neomycin resistance gene from Tn5 encoding an enzyme that confers resistance to a group of antibiotics including Geneticin. The cells are seeded in MEM supplemented with 1 mg/ml Geneticin. After 2 days, the cells are trypsinized and seeded in cloning plates in MEM supplemented with 1 mg/ml Geneticin. After about 10-14 days, single clones are trypsinized and then seeded in 6-well petri dishes or 10 ml flasks. Growing clones are then transferred to new 6-well plates. Expression of the desired gene product is analyzed, for instance, by Northern blot.

### Example 3 : Tissue distribution of HLYAL47 mRNA expression.

Northern blot analysis can be carried out to examine HLYAL47 gene expression in human tissues, using methods described by, among others, Sambrook et al., cited above. A cDNA probe containing the entire nucleotide sequence of the HLYAL47 protein (SEQ ID NO : 1) can be labeled with ³²P using the RediprimeJ DNA labeling system (Amersham Life Science, Arlington, IL), according to manufacturer's instructions. After labeling, the probe can be purified using a Chroma Spin-100J column (Clontech Laboratories, Inc.), according to manufacturer's protocol number PT1200-1. The purified labeled probe is then used to examine various human tissues for HLYAL47 mRNA expression.

Multiple Tissue Northern (MTN) blots containing various human tissues can be obtained from Clontech and examined with the labeled probe using ExpressHybJ hybridization solution (Clontech) according to manufacturer's protocol number PT1190-1. Following hybridization and washing, the blots can be mounted and exposed to film at -70°C overnight, and films developed according to standard procedures.

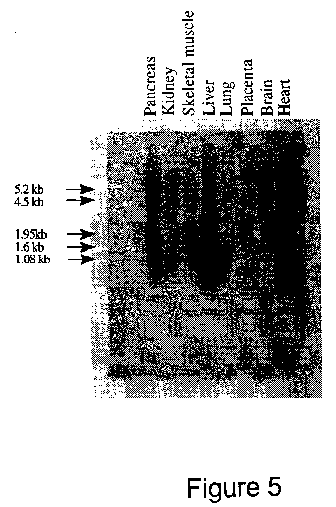

Tissue distribution is shown on figure 5. HLYAL47 mRNA expression is found mainly in liver and low mRNA expression is detected in pancreas, kidney, skelettal, placenta, brain, heart and lung.

It will be clear that the invention may be practiced otherwise than as particularly described in the foregoing description and examples.

Numerous modifications and variations of the invention are possible in light of the above teachings and, therefore, are within the scope of the appended claims.

The entire disclosure of all publications (including patents, patent applications, journal articles, laboratory manuals, books, or other documents) cited herein are incorporated by reference.

## Claims

1. An isolated polynucleotide comprising a nucleotide sequence at least 80 % identical to a nucleotide sequence selected from the group consisting of:
(a) the nucleotide sequence set forth in SEQ ID NO:1;
(b) a nucleotide sequence encoding the HLYAL47 polypeptide set forth in SEQ ID NO:2;
(c) the nucleotide sequence comprising the sequence located between nucleotide numbers 133 to nucleotide numbers 792 of SEQ ID NO:1;
(d) the nucleotide sequence encoding a polypeptide comprising amino acids from about 1 to about 219 set forth in SEQ ID NO:2;
(e) a nucleotide sequence able to hybridize with the nucleotide sequence set forth in SEQ ID NO:1 or with the cDNA clone contained in ATCC deposit number PTA-188;
(f) the nucleotide sequence encoding the HLYAL47 polypeptide expressed by the cDNA clone contained in ATCC deposit number PTA-188;
(g) a nucleotide sequence encoding the mature HLYAL47 polypeptide expressed by the cDNA clone contained in ATCC deposit number PTA-188;
(h) a nucleotide sequence complementary to any of the nucleotide sequences defined in (a), (b), (c), (d), (e), (f) or (g) above, as well as fragments, variants, derivatives, mutated forms of these nucleotide sequences.

2. Polynucleotide according to claim 1 which is DNA or RNA.

3. A DNA or RNA molecule constituting a recombinant vector, comprising an expression system which is capable of producing a HLYAL47 polypeptide when said expression system is present in a compatible host cell, wherein said DNA or RNA molecule comprises a polynucleotide according to claim 1 or 2.

4. A host cell comprising the expression system of claim 3.

5. A process for producing a HLYAL47 polypeptide comprising the steps of transforming or transfecting a host cell with an expression system according to claim 3, culturing said host cell under appropriate culture conditions so that said host cell produces HLYAL47 polypeptide, and recovering said HLYAL47 polypeptide from the culture.

6. An isolated HLYAL47 polypeptide comprising an amino acid sequence which is at least 80% identical to a sequence selected from the group consisting of:
(a) the amino acid sequence of SEQ ID NO:2;
(b) the amino acid sequence of HLYAL47 polypeptide having the complete amino acid sequence encoded by the cDNA clone contained in ATCC deposit number PTA-188;
(c) the amino acid sequence of the mature HLYAL47 polypeptide having the complete amino acid sequence encoded by the cDNA clone contained in ATCC deposit number PTA-188;as well as fragments, derivatives, variants or mutated forms of these amino acid sequences.

7. Polypeptide according to claim 6, wherein it is obtained by the process of claim 5 or by chemical synthesis.

8. An antibody, or a fragment thereof, able to recognize a HLYAL47 polypeptide according to claim 6.

9. Antibody according to claim 8 able to recognize a polypeptide comprising amino acids residues from about 19 to about 26 of SEQ ID NO:2, a polypeptide comprising amino acids residues from about 31 to about 52 of SEQ ID NO:2, a polypeptide comprising amino acids residues from about 77 to about 89 of SEQ ID NO:2, a polypeptide comprising amino acids residues from about 104 to about 115 of SEQ ID NO:2, a polypeptide comprising amino acids residues from about 120 to about 133 of SEQ ID NO:2, a polypeptide comprising amino acids residues from about 137 to about 149 of SEQ ID NO:2, a polypeptide comprising amino acids residues from about 168 to about 180 of SEQ ID NO:2.

10. A method for identifying agonists of HLYAL47 polypeptide, which comprises the steps of:
a) contacting a host cell according to claim 4 with a candidate compound; and
b) determining whether the candidate compound effects a signal generated by activation of HLYAL47.

11. An agonist identifiable by the method of claim 10.

12. An agonist according to claim 11, wherein the candidate compound is selected among compounds which present a structural, functional or both, similarity with known agonists of the CIDE family proteins, or compounds which are known to be involved in a large number of biological processes.

13. A method for identifying antagonists of HLYAL47 polypeptide, comprising the steps of:
(a) contacting host cell according to claim 4 with an agonist of HLYAL47, and
(b) determining whether the signal generated by said agonist is diminished in the presence of a candidate compound.

14. An antagonist of HLYAL47 identifiable by the method of claim 13.

15. Antagonist according to claim 14, wherein the candidate compound is selected among compounds which present a structural, functional or both, similarity with known antagonists of the CIDE family proteins, or compounds which are known to be involved in a large number of biological processes.

16. A process for diagnosing a disease or a susceptibility to a disease in a subject related to expression or activity of a HLYAL47 polypeptide comprising the steps of:
(a) determining the presence or absence of a mutation in the nucleotide sequence encoding said HLYAL47 polypeptide in the genome of said subject, and/or
(b) analyzing for the presence or amount of said HLYAL47 polypeptide expression in a sample derived from said subject.

17. Pharmaceutical composition, for prevention and/or treating in a subject a disease or susceptibility to a disease related to an under-expression or under-activity of HLYAL47 polypeptide, said composition comprising a therapeutically effective amount of at least an agonist of said HLYAL47 polypeptide or derivative or homologue thereof identified by the method of claim 10, and/or at least a HLYAL47 polypeptide according to claim 6, and/or at least a nucleotide sequence able to express *in vivo* a HLYAL47 polypeptide according to claim 6.

18. Pharmaceutical composition, for prevention and/or treating in a subject a disease or susceptibility to a disease related to an over-expression or over-activity of HLYAL47 polypeptide, said composition comprising a therapeutically effective amount of at least an antagonist of said HLYAL47 polypeptide or derivative or homologue thereof identified by the method of claim 13, and/or at least a nucleic acid molecule that inhibits the expression of the nucleotide sequence encoding HLYAL47 polypeptide and/or at least a polypeptide which competes with HLYAL47 polypeptide.

19. Pharmaceutical composition according to claim 17 or 18, comprising a recombinant vector according to claim 3, able to express *in vivo* a polypeptide according to claim 6.

20. Pharmaceutical composition according to any of claims 17 to 19, wherein it further comprises at least a pharmaceutically acceptable carrier.

21. Pharmaceutical composition according to any of claims 17 to 20, for prevention and/or treatment of cancer, hematological disorders, e.g. bone marrow failure including myelodysplastic syndromes, aplastic anemia, neutropenia and leukemia, cardiovascular diseases, liver diseases, neurodegenerative pathologies.
